(19) Europäisches Patentamt
European Patent Office
Office européen des brevets

(11) Veröffentlichungsnummer: 0 160 847
A1

(12) # EUROPÄISCHE PATENTANMELDUNG

(21) Anmeldenummer: 85104140.0

(22) Anmeldetag: 04.04.85

(51) Int. Cl.⁴: G 01 N 33/52
//C12Q1/62, C12Q1/54,
C12Q1/34

(30) Priorität: 09.04.84 DD 261784
13.03.85 DD 274087

(43) Veröffentlichungstag der Anmeldung:
13.11.85 Patentblatt 85/46

(84) Benannte Vertragsstaaten:
DE FR GB IT NL

(71) Anmelder: Akademie der Wissenschaften der DDR
Otto-Nuschke-Strasse 22/23
DDR-1086 Berlin(DD)

(72) Erfinder: Müller-Frohne, Marlies, Dr.
Waldstrasse 38
DD-1110 Berlin(DD)

(72) Erfinder: Müller, Hans-Georg, Dr.
Gürtelstrasse 11
DD-1035 Berlin(DD)

(72) Erfinder: Aumann, Jutta
Klausthaler Strasse 5
DD-1100 Berlin(DD)

(72) Erfinder: Güntherberg, Horst, Dr.
Retzbacher Weg 64
DD-1100 Berlin(DD)

(72) Erfinder: Kahrig, Erwin, Dr.
Thulestrasse 63
DD-1100 Berlin(DD)

(72) Erfinder: Kies, Jörg, Dr.
Brüderstrasse 14/207
DD-1020 Berlin(DD)

(72) Erfinder: Mohr, Peter, Prof. Dr.
Robert-Rössle-Strasse 1
DD-1115 Berlin-Buch(DD)

(72) Erfinder: Müller, Anita
Tschaikowskistrasse 29
DD-1110 Berlin(DD)

(72) Erfinder: Neumann, Barbara
Pasewalker Strasse 91
DD-1113 Berlin(DD)

(72) Erfinder: Plaschnick, Dieter
Thalheimer Strasse 42
DD-4440 Wolfen(DD)

(72) Erfinder: Scholz, Marianne
Heinrich-Roller-Strasse 16
DD-1055 Berlin(DD)

(72) Erfinder: Ullmann, Horst, Dr.
Fritz-Heckert-Strasse 26
DD-102 Berlin(DD)

(74) Vertreter: Patentanwälte Beetz sen. - Beetz jun. Timpe - Siegfried - Schmitt-Fumian
Steinsdorfstrasse 10
D-8000 München 22(DE)

(54) **Analystisches Element zur Bestimmung von Glucose, glucosehaltigen Oligosacchariden und Harnsäure.**

(57) Die Erfindung betrifft ein analytisches Element zur Bestimmung von Glucose, glucosehaltigen Oligosacchariden und Harnsäure.

Aufgabe ist es, ein analytisches Element zu schaffen, das

./...

EP 0 160 847 A1

ein definiertes Ablaufen der Nachweisreaktionen als eine farbstoffbildende Reaktionsfolge ermöglicht, wobei eine hohe Langzeitstabilität des gebildeten Farbstoffes erreicht werden soll.

Die Lösung der Aufgabe erfolgt mit einem analytischen Element, das Glucoseoxidase bzw. Uratoxidase und Peroxidase in mindestens zwei Schichten und innerhalb der Schichten in festen Konzentrationsverhältnissen und von Schicht zu Schicht als Konzentrationsgradient vorliegend homogen verteilt enthält, wobei das Glucosenachweissystem im Falle der Oligosaccharidbestimmung mit oligosaccharidspaltenden Enzymen und gegebenenfalls Mutarotase gekoppelt ist, und daß das Indikatorsystem für den $H_2O_2$–Nach- weis farbstabilisierende Mittel enthält. Die Erfindung wird in medizinischen Einrichtungen und in der Nahrungsmittelindustrie angewendet.

Analytisches Element zur Bestimmung von Glucose, glukosehaltigen Oligosacchariden und Harnsäure

Die Erfindung betrifft ein analytisches Element zur Bestimmung des Glucose- und Harnsäuregehaltes glucose- bzw. harnsäurehaltiger Flüssigkeiten mittels der gekoppelten enzymatischen Reaktion mit Glucoseoxidase bzw. Uratoxidase und Peroxidase und einem oxidierbaren Indikatorsystem, das zu einem stabilen Farbstoffprodukt führt. Weiterhin betrifft die Erfindung ein analytisches Element zur Bestimmung von glucosehaltigen Oligosacchariden, d. h. von Sacchariden, die aus zwei bis zehn Monomeren bestehen, von denen mindestens eines Glucose darstellt. Anwendungsgebiete für das erfindungsgemäße analytische Element sind die medizinischen Einrichtungen, die sich mit der Behandlung des Diabetes mellitus befassen, die Notfallmedizin, die Kontrolle von Risikofaktoren (Harnsäure), die Nahrungsmittelindustrie, die Getränkeindustrie und die mikrobiologische Industrie.

Im Rahmen der Rationalisierung und Einsparung von Reagentien werden die Bestimmung von Glucose und Harnsäure in zunehmendem Maße als Schnelltests mittels Teststreifen durchgeführt. Das Prinzip der Nachweisreaktion beruht vorwiegend auf der enzymatischen Oxidation der Glucose bzw. der Harnsäure durch Glucoseoxidase bzw. Uratoxidase zu Wasserstoffperoxid und der Bestimmung des gebildeten $H_2O_2$ durch eine weitere enzymatische Reaktion mittels Peroxidase und einem oxidierbaren Indikatorsystem zu einem Farbstoff, dessen Konzentration entweder quantitativ durch photometrische bzw. reflektometrische Messung

oder qualitativ durch visuellen Farbvergleich mit einer vorgegebenen Farbtafel erfaßt wird. Dazu ist es erforderlich, daß über einen weiten Konzentrationsbereich der zu bestimmenden Substanzen die Nachweisreaktion so gelenkt wird, daß kein Farbstoffgemisch, sondern ein stabiles, definiertes Farbstoffprodukt entsteht (DE-OS 29 32 973, DE-OS 27 17 817, DE-AS 25 32 918, DE-AS 23 32 760). In den genannten Erfindungsbeschreibungen werden Teststreifen beschrieben, die einen zum Teil komplizierten Schichtaufbau zeigen, der besonders aufwendige Herstellungstechnologien erfordert. Des weiteren sind die beschriebenen Streifen entweder nur für visuelle oder nur für metrische Auswertung geeignet, die außerdem nur mit zusätzlichen, speziellen Geräteentwicklungen durchgeführt werden kann.
Bei der in dem DDR-WP 135 243 beschriebenen Lösung besteht der entscheidende Nachteil darin, daß der gebildete Farbstoff nur über einen engen Konzentrationsbereich den zu bestimmenden Substanzen proportional ist und kein einheitliches Reaktionsprodukt entsteht.
Außerdem zeigt der Farbstoff eine ungenügende Zeitstabilität und Diffusionsfestigkeit.
Hinzu kommt, daß die Teststreifen einen eingeschränkten Gebrauchswert durch ihre geringe mechanische Oberflächenfestigkeit zeigen, insbesondere beim Abwischen des aufgetragenen Blutes.
Die analytische Bestimmung von glucosehaltigen Oligosacchariden erfolgt gegenwärtig fast ausschließlich enzymatisch. Die noch gelegentlich angewendete polarimetrische Bestimmung erfordert klare Lösungen. Für die enzymatische Bestimmung läßt man ein geeignetes Enzym zum Zwecke der Spaltung in Monosaccharide auf die Probe einwirken. Das entstehende Monosaccharid (Glucose) wird anschließend ebenfalls enzymatisch bestimmt. Die bevorzugte

Nachweisreaktion für Glucose ist die Zunahme der Extinktion von NADPH bzw. NADH im langwelligen UV-Licht. Folgende Reaktionen laufen beispielsweise dabei ab:

Maltose $\xrightarrow{\text{Maltase}}$ 2 Glucose

Glucose $\xrightarrow[\text{ATP}]{\text{Hexokinase}}$ Glucose-6-phosphat + ADP

Glucose-6-phosphat $\xrightarrow[\text{NADP}]{\text{Glucose-6-phosphat-Dehydrogenase}}$ Gluconat-6-phosphat + NADPH

Saccharose $\xrightarrow{\text{Invertase}}$ Glucose + Fructose (Glucosebestimmung wie oben)

Lactose $\xrightarrow{\text{Lactase}}$ Galactose + Glucose

Galactose $\xrightarrow{\text{Galactose-Dehydrogenâse}}$ Galactonolacton+NADH

Alle diese Methoden sind sogenannte Küvettentests. Für ihre Durchführung ist ein gut ausgestattetes Labor notwendig, wie z. B. das Vorhandensein eines UV-Spektralphotometers. Außerdem erfordern diese Methoden einen größeren Zeitaufwand und einen höheren Verbrauch an Chemikalien, insbesondere an Enzymen. Mit Hilfe von Küvettentests ist eine unkomplizierte schnelle Analyse unter Produktionsbedingungen, d. h. in unmittelbarer Nähe der Produktionsanlage nicht möglich.

Das Ziel der Erfindung besteht darin, die Bestimmung von Glukose, glukosehaltigen Oligosacchariden und Harnsäure ökonomisch günstig und mit hoher Genauigkeit auch durch nicht geschultes Personal durchzuführen, wobei die Auswertung im größeren zeitlichen Abstand nach der Auftragung der Probe möglich sein soll.

Der Erfindung liegt die Aufgabe zugrunde, ein neues, mehrschichtig aufgebautes, analytisches Element zu schaffen, das ein definiertes Ablaufen der Nachweisreaktion

von Glucose, glukosehaltigen Oligosacchariden und Harnsäure als eine farbstoffbildende Reaktionsfolge ermöglicht, eine Langzeitstabilität des gebildeten Farbstoffes und einen Oberflächenschutz der obersten Reagenzschicht gewährleistet.

Die Aufgabe wird erfindungsgemäß durch ein neues, mehrschichtig aufgebautes, analytisches Element zur Bestimmung von Glucose, glukosehaltigen Oligosacchariden und Harnsäure in Flüssigkeiten auf der Grundlage der Wirkung $H_2O_2$-bildender und $H_2O_2$-übertragender Enzyme mit lichtdurchlässigen und lichtundurchlässigen Trägermaterialien und mit Gelatine als Bindemittel für Nachweis- und Indikatorreagenzien wie Glucoseoxidase bzw. Uratoxidase, Peroxidase und Benzidin- oder Phenylendiaminderivaten als Farbstoffvorstufen gelöst. Die Erfindung ist dadurch gekennzeichnet, daß Glucoseoxidase bzw. Uratoxidase und Peroxidase in mindestens zwei Schichten und innerhalb der Schichten in festen Konzentrationsverhältnissen und von Schicht zu Schicht als Konzentrationsgradient vorliegend, homogen verteilt sind, wobei das Glucosenachweissystem gegebenenfalls mit oligosaccharidspaltenden Enzymen und gegebenenfalls Mutarotase gekoppelt ist, und daß das Indikatorsystem für den $H_2O_2$-Nachweis farbstabilisierende Mittel enthält.

Für den Glucose- bzw. Harnsäurenachweis befinden sich auf dem Träger mindestens zwei Reagenzschichten, gegebenenfalls eine Schutzschicht und zwischen der unteren und oberen Reagenzschicht gegebenenfalls weitere Reagenzschichten. Die Reagenzschichten enthalten Glucoseoxidase bzw. Uratoxidase und Peroxidase in definierten Konzentrationen und Konzentrationsverhältnissen und Farbfixiermittel, wobei die Konzentration der Enzyme von der unteren zur oberen Schicht abnimmt und das Konzentrationsverhältnis von Glucose- bzw. Uratoxidase zu Peroxidase innerhalb der Schichten kleiner oder gleich 1 ist.

Die obere Reagenzschicht weist eine Konzentration sowohl an Glucoseoxidase bzw. Uratoxidase als auch an Peroxidase auf, die 1,5 - 85 % geringer ist als die Konzentration der genannten Enzyme in der auf dem Träger haftenden unteren Schicht. Das Konzentrationsverhältnis von Glucoseoxidase bzw. Uratoxidase zu Peroxidase in der oberen Reagenzschicht soll 1 : 1 bis 1 : 100 und in der unteren Schicht 1 : 1 bis 1 : 80 betragen. Zwischen der oberen und der unteren Reagenzschicht befinden sich gegebenenfalls weitere Reagenzschichten, deren Konzentrationen und Konzentrationsverhältnisse an den genannten Enzymen sich innerhalb der Werte der oberen und unteren Schicht bewegen. Auf der Oberfläche der oberen Reagenzschicht ist eine Schutzschicht vorhanden, die im wesentlichen aus Gelatine mit höherem Vernetzungsgrad besteht. Die Schichtdickenverhältnisse der Schutzschicht und der oberen Reagenzschicht betragen 1 : 1 bis 1 : 25 und der oberen und unteren Reagenzschicht 2 : 1 bis 1 : 5 bei einer Gesamtschichtdicke des erfindungsgemäßen analytischen Elementes von maximal 50 /um. Die erfindungsgemäß eingesetzten Farbfixiermittel, die die diffusionsfähige Farbstoffvorstufe nach Oxidation diffusionsfest machen und chemisch stabilisieren, sind Verbindungen der allgemeinen Formel I, II, III, IV oder V(siehe Formelblatt), wobei sich insbesondere die Verbindungen eignen, deren Kupplungsfunktion durch Substituenten wie Alkyl- oder Alkoxy-Gruppen blockiert ist. Das Farbfixiermittel liegt im Vergleich zur Farbstoffvorstufe in allen Schichten sowohl im Unter- als auch im Oberschuß vor.

Als Trägermaterialien werden vorzugsweise transparente Kunststoffolien, z. B. Celluloseacetatfolien, für die Durchlichtbetrachtung und für die Auflichtbetrachtung weiße undurchsichtige Unterlagen aus synthetischem oder natürlichem Faservlies, z. B. photographische Papiere, eingesetzt. Allerdings darf das Trägermaterial die Nachweisreaktion nicht beeinflussen. Als Bindemittel für die oben erwähnten Ingredenzien wird Gelatine in geeigneter Vernetzung, die teilweise durch Gelatineersatzstoffe ausgetauscht werden kann, benutzt. Nach Einbringung der Ingredenzien, wie Farbstoffvorstufen, die erfindungsgemäßen Farbfixiermittel, grenzflächenaktive Stoffe, Weichmacher und die die Nachweisreaktion katalysierenden Enzyme in den erfindungsgemäßen Konzentrationsverhältnissen, erfolgt der Auftrag der Schichten in der Art der Herstellung photographischer Filme. Die Anzahl der Schichten, die auf dem Träger aufgebracht werden, und die absoluten Konzentrationen der Enzyme Glucoseoxidase, Uratoxidase und Peroxidase werden im wesentlichen durch den Gehalt an Glucose und Harnsäure in den zu untersuchenden Flüssigkeiten und die spezifischen Aktivitäten der Enzyme bestimmt. In Abhängigkeit vom jeweiligen Einsatzgebiet der analytischen Elemente sind die absoluten Enzymkonzentrationen in weiten Grenzen variierbar. Allerdings kommt den erfindungsgemäßen relativen Konzentrationsverhältnissen innerhalb einer Schicht und zwischen den Reagenzschichten eine besonders große Bedeutung zu. Es hat sich gezeigt, daß durch Schaffung von Konzentrationsgradienten zwischen der unteren und der oberen Schicht und durch Abstufung der Konzentration von $H_2O_2$-bildenden Enzymen (Glucoseoxidase bzw. Uratoxidase) gegenüber $H_2O_2$-übertragenden Enzymen (Peroxidase) in einer Reagenzschicht ein definierter Reaktionsablauf für die enzymatische Umsetzung der nachzuweisenden Substanz und die anschließenden Folgereaktionen der enzymatisch katalysierten Oxidation der Farbstoffvorstufe und der nachfolgenden Stabilisierungsreaktion des Farbproduktes erreicht wird. In Abb. 1a ist der Extinktions-Zeit-Verlauf für bisher bekannte Lösungen dargestellt, der zu Eichkurven führt, wie sie Abb. 2a zeigen.

Die Abbildungen 1b und 2b stellen den Extinktions-Zeit-Verlauf bzw. die dazugehörige Eichkurve bei Verwendung des erfindungsgemäßen analytischen Elementes dar.
Der geforderte Konzentrationsgradient an Enzymen innerhalb des analytischen Elementes wird durch das Aufbringen mehrerer Schichten realisiert.
Einen besonders günstigen Verlauf des Gradienten erreicht man je nach Einsatzgebiet durch Optimierung von Reagenzschichtdicke, Anzahl der Schichten und absoluter Enzymkonzentration. In der Reagenzschicht muß erfindungsgemäß die Menge an Peroxidase größer sein als die Menge an Glucoseoxidase bzw. Uratoxidase. Es ist jedoch durchaus möglich, daß bei mehrschichtigem Material in einer Reagenzschicht das Konzentrationsverhältnis 1 : 1 betragen kann. Besonders günstig ist ein 3 - 50facher Überschuß an Peroxidase. Dieser Überschuß beschleunigt nach Umsetzung von Glucose durch Glucoseoxidase bzw. Harnsäure durch Uratoxidase zu Wasserstoffperoxid in besonderem Maße die Oxidation der Farbstoffvorstufe und die Reaktion des Oxidationsprodukts mit dem Farbfixierungsmittel zu einem einheitlichen Farbprodukt.
Für das Erzeugen einer Oberflächenschutzschicht auf dem Reagenzschichtsystem aus stärker vernetzter Gelatine bieten sich mehrere Möglichkeiten an. Das Auftragen der Gelatineschicht, die das Vernetzungsmittel enthält, nach den üblichen Verfahren der photographischen Industrie ist eine dieser Möglichkeiten. Eine zweite Variante ist das Aufsprühen von Gelatine-Vernetzungsmittel-Lösungen und eine dritte das Aufsprühen einer reinen Vernetzungsmittel-Lösung auf die obere Reagenzschicht. Mit Hilfe der letztgenannten Methode sind besonders dünne Schichten herstellbar. Um das Benetzungsverhalten der zu untersuchenden Flüssigkeiten auf der Schutzschicht günstig zu gestalten, können der Schutzschicht grenzflächenaktive Stoffe zugesetzt werden, die die Verteilung der Probe bewirken, bevor der Quellvorgang einsetzt.

Das erfindungsgemäße analytische Element zeichnet sich dadurch aus, daß es sowohl metrisch ausgewertet werden kann, da die Extinktion des gebildeten Farbproduktes über einen breiten Konzentrationsbereich dem zu bestimmenden Stoff, vorzugsweise 1,5 - 40 oder 30 - 300 mMol/l Glucose bzw. 0,15 bis 0,7 mMol/l Harnsäure, proportional ist, als auch die Stabilität des gebildeten Farbstoffes einen visuellen Farbvergleich mit einer geeigneten vorgegebenen Farbtafel ermöglicht. Damit ist eine Auswertung ohne ein Meßgerät möglich, bzw. die Messung kann zu einem späteren Zeitpunkt nachgeholt werden, was eine breite Anwendung, besonders für Reihenuntersuchung oder zur Selbstkontrolle der Diabetiker, ermöglicht.

Die Bestimmung der Oligosaccharide erfolgt erfindungsgemäß mit einem modifizierten analytischen Element, das ein Testfeld auf einer transparenten Unterlage aufweist, in dem in mehreren Schichten die notwendigen Chemikalien für den Oligosaccharidnachweis eingearbeitet sind und in dem nach Auftragen der flüssigen oligosaccharidenthaltenden Probe ein Reaktionsmechanismus in Gang gesetzt wird, der im Ergebnis eine dem Oligosaccharidgehalt proportionale Färbung ergibt, die sowohl photometrisch als auch durch Benutzung eines Komparators quantitativ auswertbar ist.

Der Aufbau des analytischen Elementes soll im folgenden näher erläutert werden: Auf dem oben beschriebenen Glukosenachweissystem ist zusätzlich ein oligosaccharidspaltendes System eingearbeitet bzw. gekoppelt.

Es enthält die spaltenden Enzyme und ggf. das Enzym Mutarotase, das für die Einstellung des natürlichen Verhältnisses zwischen $\alpha$- und ß-Glucose notwendig ist für den Fall, daß nur $\alpha$-Glucose bei der Spaltung entsteht, z. B. bei der Einwirkung von Invertase auf Rohrzucker.

Die absoluten Konzentrationen der Reagenzien, insbesondere der Enzyme, richten sich nach dem jeweiligen Verwendungszweck des analytischen Elementes. Höher konzentrierte Proben erfordern höhere Enzymkonzentrationen bzw. ein Anpassen der Probenlösungen durch Verdünnen. Wesentlich sind jedoch die Konzentrationsverhältnisse der Enzyme in den Schichten. Die Konzentration an oligosaccharidspaltendem Enzym entspricht größenordnungsmäßig der Konzentration an GOD. Dementsprechend liegt auch die einzusetzende Konzentration an Mutarotase fest, wenn im Ergebnis der Spaltung nur $\alpha$-Glucose entsteht.
Das oligosaccharidspaltende System wird zweckmäßigerweise in die obere Schicht des Glucosenachweissystems eingearbeitet. In einer anderen Ausführungsform der Erfindung wird eine zusätzliche Schicht auf das Glucosenachweissystem aufgebracht, die das oligosaccharidspaltende System enthält. Diese Schicht kann ebenfalls aus einem quellfähigen, filmbildenden Polymer bestehen und wird nach bekannter Technologie aufgebracht. In einer dritten Variante kann diese Schicht auch aus einem saugfähigen Material, wie z. B. Filterpapier oder Vlies, bestehen, in das durch Tränkung und anschließendes Trocknen das oligosaccharidspaltende System eingearbeitet wurde.
Die transparente Unterlage liegt als geschnittener Streifen vor, auf dem an einem Ende das mehrschichtige Testfeld aufgebracht ist. Der Streifen mit dem Testfeld kann mit weiteren Materialien zur besseren Handhabbarkeit kombiniert werden. Beispielsweise ermöglicht eine auf der Rückseite angebrachte weiße Reflexionsschicht Auswertungen im Auflicht.
Bei der Benutzung der analytischen Elemente wird die zu untersuchende Probenflüssigkeit mit der obersten Schicht in Kontakt gebracht. Das erfolgt zweckmäßigerweise durch Auftropfen, Einwirkenlassen und ggf. Entfernen der Tropfenreste. Das polymere Bindemittel quillt, und dadurch sind innerhalb der Schicht Transportvorgänge durch

Diffusion möglich. In der obersten Schicht wird das zu bestimmende Oligosaccharid durch die anwesenden Enzyme zu Glucose gespalten und ggf. durch Mutarotase das natürliche Verhältnis von α-Glucose zu ß-Glucose eingestellt. Beim Diffundieren der Glucose in die darunterliegenden Schichten bzw. gleich bei der Entstehung erfolgt der an sich bekannte enzymatische Nachweis der Glucose durch Umsetzung mit Glucoseoxidase zu $H_2O_2$ und die Oxidation einer Farbstoffvorstufe - in diesem Fall o-Tolidin - unter katalytischer Mitwirkung von Peroxidase zu einem Farbstoff, der dann im Durchlicht bzw. Auflicht photometrisch oder mit einem Komparator ausgemessen wird.
Folgende Enzyme, die bei ihrer Einwirkung auf Oligosaccharide Glucose freisetzen, können einzeln, aber auch im Gemisch Bestandteil des analytischen Elementes sein:

| | |
|---|---|
| Glucoamylase | (EC 3.2.1.3) |
| Maltase | (EC 3.2.1.20) |
| Cellobiase | (EC 3.2.1.21) |
| Invertase | (EC 3.2.1.26) |
| Lactase | (EC 3.2.1.23) |

Als quellfähiges polymeres Bindemittel wird in der Regel Gelatine verwendet.
Das analytische Element arbeitet schnell und mit großer Präzision. Es erlaubt ohne großen Aufwand, den Gehalt an niedermolekularen glucosehaltigen Sacchariden zu bestimmen, so daß beispielsweise eine einfache Prozeßkontrolle möglich ist, ohne ein größeres chemisches Labor betreiben zu müssen. Das hat vor allen Dingen in kleinen Betrieben der Lebensmittel- und Getränkeindustrie Bedeutung.

Die Erfindung wird im folgenden anhand von Ausführungsbeispielen näher erläutert.

Beispiel 1

In 100 ml einer 6 %igen Gelatinelösung, die auf einen pH-Wert zwischen 4,5 - 4,9 eingestellt ist und entsprechende Zusätze an in der Filmtechnologie üblichen Emulgatoren und vernetzenden Reagenzien enthält, werden 10 mMol/l o-Tolidinhydrochlorid als Farbbildner und 7,5 mMol/l des diffusionsfesten Farbfixiermittels 3-(N-1-Oxynaphthoyl)-4-(N-methyl-N-octadecyl)-benzolsulfonsäure gelöst. Zum Aufbringen der 1. Schicht auf eine Celluloseacetatfolie als Trägerschicht werden der Begußlösung 400 Units Glucoseoxidase und 4 800 Units Peroxidase zugesetzt. Auf diese Schicht wird eine weitere Schicht aufgetragen, die 300 Units Glucoseoxidase und 2 500 Units Peroxidase enthält. Als 3. Schicht wird eine stärker vernetzte Gelatineschicht, 200 Units Glucoseoxidase und 1 500 Units Peroxidase enthaltend, aufgetragen. Die Gesamtschichtdicke soll ca. 25 $\mu$m betragen. Es werden ca. 0,3 $m^2$ beschichtete Reagenzfolie erhalten, mit der Glucosebestimmungen im Blut oder Serum im Konzentrationsbereich von ca. 3 - 40 mMol/l durchgeführt werden können.

Beispiel 2

Zur Bestimmung von Glucose im Konzentrationsbereich von 1,5 - 6 mMol/l werden,wie in Beispiel 1 beschrieben, zwei Reagenzschichten auf eine weiße fotopapierähnliche Unterlage aufgetragen. 100 ml Begußlösung für die 1. Schicht enthalten 1 000 Units Glucoseoxidase und 8 000 Units Peroxidase, für die 2. Schicht 600 Units Glucoseoxidase und 5 000 Units Peroxidase. Die Konzentration an o-Tolidin und 1-Methoxy-2-nonadecyl-naphthalinsulfonsäure-(4) beträgt 15 mMol/l. Die Gesamtschichtdicke soll ca. 20 $\mu$m betragen. Das analytische Element kann nach Reaktion mit glucoseenthaltenden Flüssigkeiten durch Farbvergleich mit einer vorgegebenen Farbtafel visuell oder durch Reflexionsmessung metrisch ausgewertet werden.

Beispiel 3

Zur Bestimmung von Glucose in Fermentationslösungen im Konzentrationsbereich von 30 - 300 mMol/l wird ein analytisches Element von der Gesamtschichtdicke ca. 40 /um, bestehend aus 6 Schichten, hergestellt, die folgende Konzentrationen an Enzymen, o-Tolidin und dem Farbfixiermittel Na-Salz der 1-(3-Methoxymethyl-5-methylsulfonsäure-benzyl)-2-methoxy-naphthalinsulfonsäure-(6) enthalten.

| Schicht | Glucoseoxidase (Units) | Peroxidase (Units) | o-Tolidin mMol/l | Farbfixiermittel mMol/l |
|---|---|---|---|---|
| 1 | 3 000 | 10 000 | 15 | 10 |
| 2 | 2 500 | 10 000 | 15 | 10 |
| 3 | 1 000 | 5 000 | 100 | 7,5 |
| 4 | 500 | 5 000 | 10 | 7,5 |
| 5 | 250 | 3 000 | 7,5 | 7,5 |
| 6 | 100 | 2 000 | 7,5 | 5,0 |

Beispiel 4

Zur Herstellung eines analytischen Elementes für die Bestimmung von Harnsäure im Serum werden 100 ml Begußlösung hergestellt, indem in 50 ml einer 16 %igen Gelatinelösung unter Luftausschluß 1,2 mMol/l p-Diethylen-phenylendiaminsulfat und 2,5 mMol/l 1-(Phenylsulfonsäure-(4))-3-octadecyl-4-äthyl-pyrazolon-(5) eingetragen werden. Durch Zugabe von 90 Units Uratoxidase und 5 000 Units Peroxidase in einer Boratpufferlösung vom pH 8,5 wird die Begußlösung auf 100 ml ergänzt. Mit dieser Lösung überzieht man den Schichtträger. Nach dem Trocknen dieser Schicht wird eine weitere Schicht aufgetragen, die 60 Units Uratoxidase und 3 000 Units Peroxidase enthält. Das Reagenzenthaltende Schichtsystem wird mit einer 5 /um starken Schutzschicht aus stark vernetzter Gelatine überzogen. Die Gesamtschichtdicke soll 30 /um betragen.

Beispiel 5

In 20 ml Acetatpuffer (pH 4,9, Ionenstärke 0,1) sind o-Tolidinhydrochlorid und 1-/4-Phenoxy-phenylsulfonsäure-(3)/-3-octadecyl-4-äthyl-pyrazolon-(5) in einer Konzentration von 10 mMol bzw. 7 mMol/l gelöst, des weiteren 1 000 Units Peroxidase und 60 Units GOD. Dieser Lösung werden 1,5 g Gelatine zugesetzt, z. B. dadurch, daß in 10 ml des genannten Puffers bei etwa 40°C die Gelatine aufgeschmolzen und diese Lösung mit den anderen Bestandteilen vermischt wird. Die so erhaltene Gelatine-Reagenzlösung wird auf eine transparente Unterlage (z. B. Acetat- bzw. Polyesterfolie) vergossen. Nach Trocknung wird auf diese Schicht eine weitere Reaktionsschicht wie folgt aufgetragen: etwa 2 ml einer 10 %igen Gelatine-Lösung, in der 6 Units Glucoseoxidase und das obengenannte Farbfixiermittel in einer Konzentration von 7 mmol/l enthalten sind, werden auf ca. 50 $cm^2$ der 1. Schicht aufgetragen. Dieser 2-Schichtenfilm kann zum Nachweis von Glucose unter Verwendung einer Eichkurve benutzt werden, wobei die Auswertung visuell oder fotometrisch bei 600 - 650 nm erfolgen kann.

Beispiel 6

Einer 7 %igen Gelatinelösung werden Glucoseoxidase (80 mg/l) und Peroxidase (400 mg/l) sowie o-Tolidin-hydrochlorid (3 g/l) zugesetzt. Diese Lösung wird auf eine transparente Unterlage aufgetragen (z. B. Ac- oder Polyesterfolie). Nach der Verfestigung der Gelatine wird auf diese Gelatineschicht eine weitere Gelatinelösung aufgetragen, die außer Glucoseoxidase (40 mg/ml), Peroxidase (400 mg/l) und o-Tolidin-hydrochlorid (3 g/l) noch Glucoamylase (100 mg/l) enthält. Nach Festwerden auch dieser 2. Gelatineschicht kann die Probelösung aufgetragen werden. Man läßt diese eine definierte Zeit einwirken (z. B.

1 Minute). Nach weiteren 10 Minuten kann die Konzentration photometrisch oder durch visuellen Farbvergleich ermittelt werden.
Erfaßt werden Maltose, Maltotriose und längerkettige Oligosaccharide, die aus α-1,4-verknüpfter Glucose bestehen.

Beispiel 7

Wie Beispiel 6, jedoch ist die Glucoamylase nicht in der 2. Gelatineschicht enthalten, sondern befindet sich in einer gesonderten 3. Gelatineschicht (= oberste Schicht).

Beispiel 8

Wie Beispiel 6, jedoch ist die Glucoamylase nicht in der 2. Gelatineschicht enthalten, sondern befindet sich in einem aus Zellulose bestehenden Material, das auf die Gelatineschicht aufgelegt und nach der Einwirkung der Proben entfernt wird.

Beispiel 9

Wie Beispiel 6, jedoch fehlt der 2. Gelatineschicht die Glucoamylase. An ihre Stelle treten Invertase (50 mg/l) und Mutarotase (25 mg/l). Erfaßt wird damit Saccharose.

Beispiel 10

Wie Beispiel 9, jedoch sind die Invertase (50 mg/l) und Mutarotase (25 mg/l) nicht in der 2. Gelatineschicht enthalten, sondern befinden sich in einer gesonderten 3. Gelatineschicht (= oberste Schicht).

Beispiel 11

Wie Beispiel 9, jedoch sind die Invertase (50 mg/l) und Mutarotase (25 mg/l) nicht in der 2. Gelatineschicht, sondern befinden sich in einem aus Zellulose bestehenden

Material, das auf die Gelatineschicht aufgelegt und nach der Einwirkung der Probe entfernt wird.

Beispiel 12

Wie Beispiel 6, jedoch fehlt der 2. Gelatineschicht die Glucoamylase, An ihre Stelle treten Lactase (60 mg/l) und Mutarotase (15 mg/l). Erfaßt wird damit Lactose.

Beispiel 13

Wie Beispiel 12, jedoch sind die Lactase (60 mg/l) und Mutarotase (15 mg/l) nicht in der 2. Gelatineschicht enthalten, sondern befinden sich in einer gesonderten 3. Gelatineschicht (= oberste Schicht).

Beispiel 14

Wie Beispiel 12, jedoch befinden sich die Lactase (60 mg/l) und die Mutarotase (15 mg/l) in einem aus Zellulose bestehenden Material, das auf die Gelatine aufgelegt und nach der Einwirkung der Probe entfernt wird.

**Formelblatt**

(I)

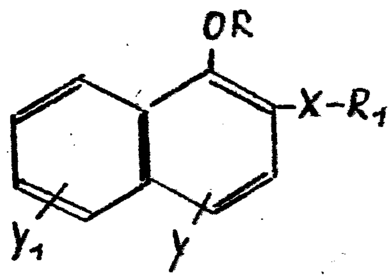

wobei

R = H- oder Alkylrest

X = $-CH_2-$; $-\underset{\|}{\overset{}{C}}-NH$; $-C-N-$ (mit =O am C, $R_2$ am N)

$R_1$ = Alkylrest mit 4 - 22 Kohlenstoffatomen

Y; $Y_1$ = H-; $-SO_3H$; -COOH, $-SO_2Me$; -COOMe - Alkyl, wobei wenigstens Y oder $Y_1$ eine anionische Gruppe der aufgeführten Struktur darstellen muß

Me = $Na^+$, $K^+$, $NH_4^+$

$R_2$ = Alkylrest mit 1 - 22 Kohlenstoffatomen,

(II)

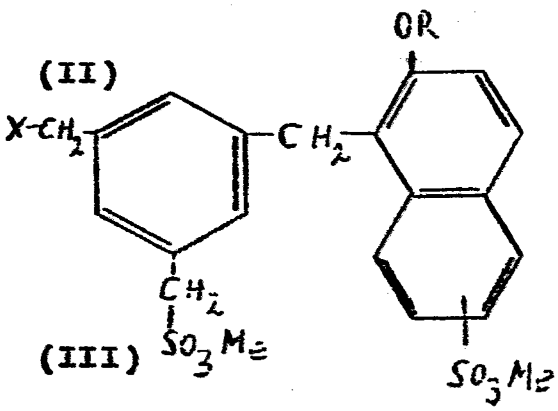

(III)

wobei

R = Alkylrest mit 1 - 8 C-Atomen oder H

Me = $H^+$; $Na^+$, $K^+$, $NH_4^+$

X = $-SO_3Me$ oder -OR,

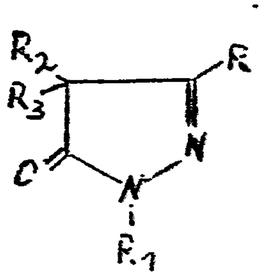

wobei

R = Alkylrest mit 4 - 22 C-Atomen, -NMR; $-NH-\underset{\underset{O}{\|}}{C}-R$

$R_1$ = substituierter Phenylrest der über $-SO_3Me$ oder -COOMe-Gruppen verfügt

$R_2$, $R_3$ = H-; $-CH_3$; $-CH{<}^{CH_3}_{CH_3}$,

0160847

(IV)

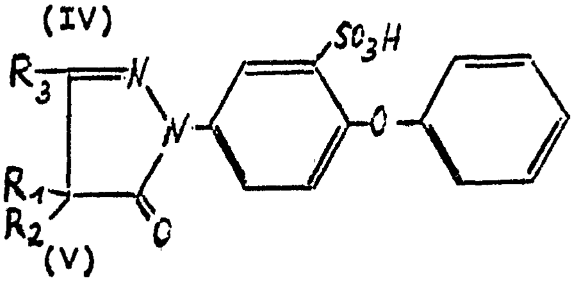

wobei

$R_1$, $R_2$ = H (oder ein Alkylrest

$R_3$ = langkettiger Alkyl-substituent

(V)

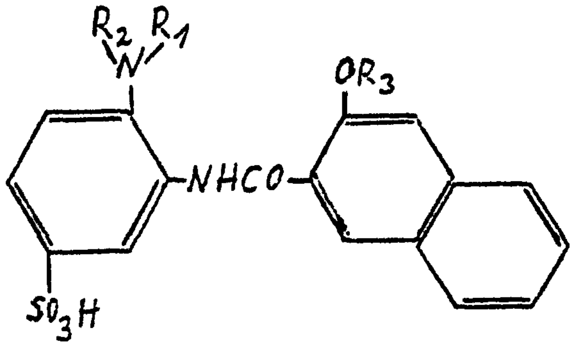

wobei

$R_1$, $R_2$ = Alkyl (1 - 20 C-Atome)

$R_3$ = Alkyl (1 - 6 C-Atome)

0160847

## Ansprüche

1. Analyticshes Element zur Bestimmung von Glucose, glucosehaltigen Oligosacchariden und Harnsäure auf der Grundlage der Wirkung $H_2O_2$-bildender und $H_2O_2$-übertragender und gegebenenfalls oligosaccharidspaltender Enzyme, mit lichtdurchlässigen und lichtundurchlässigen Trägermaterialien, mit Gelatine als Bindemittel für Nachweis- und Indikatorreagenzien wie Enzymen und Benzidin- oder Phenylendiaminderivaten als oxidierbare Farbstoffvorstufen, gekennzeichnet dadurch, daß Glucoseoxidase bzw. Uratoxidase und Peroxidase in mindestens zwei Schichten und innerhalb der Schichten in festen Konzentrationsverhältnissen und von Schicht zu Schicht als Konzentrationsgradient vorliegend homogen verteilt sind, wobei das Glucosenachweissystem [im Falle der Oligosaccharidbestimmung] mit oligosaccharidspaltenden Enzymen und gegebenenfalls Mutarotase gekoppelt ist, und daß das Indikatorsystem für den $H_2O_2$-Nachweis farbstabilisierende Mittel enthält.

2. Analytisches Element nach Anspruch 1, gekennzeichnet dadurch, daß sich auf dem Träger mindestens zwei Reagenzschichten, gegebenenfalls eine Schutzschicht und zwischen der unteren und oberen Reagenzschicht gegebenenfalls weitere Reagenzschichten befinden, daß die Reagenzschichten Glucoseoxidase bzw. Uratoxidase und Peroxidase in definierten Konzentrationen und Konzentrationsverhältnissen und Farbfixiermittel enthalten, wobei die Konzentration der Enzyme von der unteren zur oberen Schicht abnimmt und das Konzentrationsverhältnis von Glucoseoxidase bzw. Uratoxidase zu Peroxidase innerhalb der Schichten kleiner oder gleich 1 ist.

3. Analytisches Element nach Anspruch 1 und 2, gekennzeichnet dadurch, daß auf dem Träger mindestens zwei

Reagenzschichten vorhanden sind, wobei die obere Schicht eine Konzentration sowohl an Glucoseoxidase bzw. Uratoxidase als auch an Peroxidase aufweist, die 1,5 - 85 % geringer ist als die Konzentration der genannten Enzyme in der auf dem Träger haftenden unteren Schicht, daß das Konzentrationsverhältnis von Glucoseoxidase bzw. Uratoxidase zu Peroxidase in der oberen Reagenzschicht 1 : 1 bis 1 : 100 und in der unteren Schicht 1 : 1 bis 1 : 80 beträgt, daß sich zwischen der oberen und unteren Reagenzschicht gegebenenfalls weitere Reagenzschichten befinden, deren Konzentrationen und Konzentrationsverhältnisse sich innerhalb der Werte der oberen und unteren Schicht bewegen, daß auf der Oberfläche der oberen Reagenzschicht eine Schutzschicht vorhanden ist, die im wesentlichen aus Gelatine mit höherem Vernetzungsgrad besteht, daß die Schichtdickenverhältnisse der Schutzschicht und der oberen Reagenzschicht 1 : 1 bis 1 : 25 und der oberen und unteren Reagenzschicht 2 : 1 bis 1 : 5 beträgt und daß die Gesamtdicke der Schichten maximal 50 /um aufweist.

4. Analytisches Element nach Anspruch 1 - 3, gekennzeichnet dadurch, daß das Farbfixiermittel eine Verbindung der allgemeinen Formeln I, II, III, IV und/oder V ist (vgl. Formelblatt).

5. Analytisches Element nach Anspruch 1 - 4, gekennzeichnet dadurch, daß das Farbfixiermittel in allen Schichten im Vergleich zur Farbstoffvorstufe sowohl im Unter- als auch im Überschuß vorliegt.

6. Analytisches Element nach Anspruch 1 - 5, gekennzeichnet dadurch, daß das Konzentrationsverhältnis von Peroxidase zu Glucoseoxidase bzw. Uratoxidase in der oberen Schicht gegen Null geht, jedoch in der unteren Schicht 1 : 1 bis 80 : 1 beträgt und daß in der oberen Schicht sich keine oxidierbare Farbstoffvorstufe befindet.

7. Analytisches Element nach Anspruch 1 - 6, gekennzeichnet dadurch, daß das Glucosenachweissystem zusätzlich ein oder mehrere oligosaccharidspaltende Enzyme enthält oder daß diese Enzyme in einem polymeren quellfähigen Bindemittel oder in einem saugfähigen Material als zusätzliche Schicht auf das Glucosenachweissystem aufgetragen sind, wobei gegebenenfalls Mutarotase zugesetzt wird.

8. Analytisches Element nach Anspruch 7, gekennzeichnet dadurch, daß als oligosaccharidspaltende Enzyme

| | |
|---|---|
| Glucoamylase | (EC 3.2.1.3) |
| Maltase | (EC 3.2.1.20) |
| Cellobiase | (EC 3.2.1.21) |
| Invertase | (EC 3.2.1.26) |
| Lactase | (EC 3.2.1.23) |

zugesetzt werden.

9. Analytisches Element nach Anspruch 7 und 8, gekennzeichnet dadurch, daß als saugfähiges Material Filterpapier und Vlies verwendet werden.

10. Analytisches Element nach Anspruch 7 - 9, gekennzeichnet dadurch, daß als polymeres quellfähiges Bindemittel insbesondere Gelatine verwendet wird.

1/1

0160847

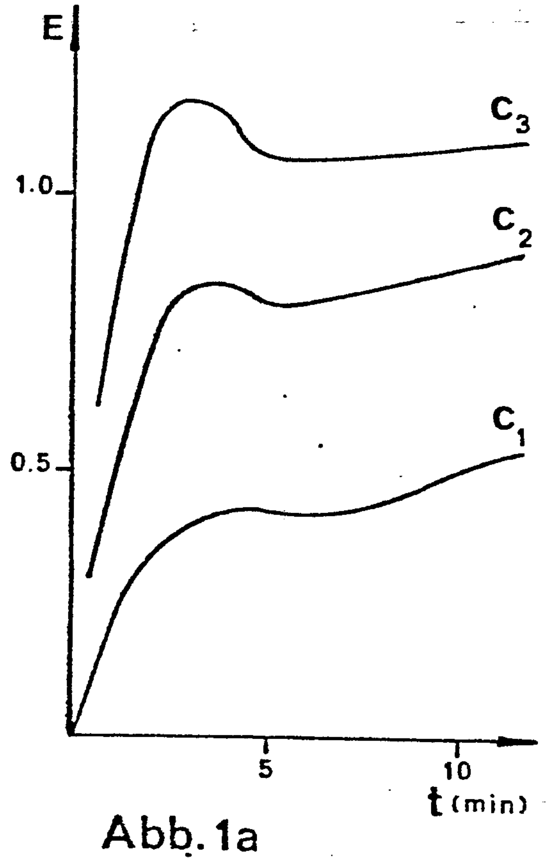


Abb. 1a

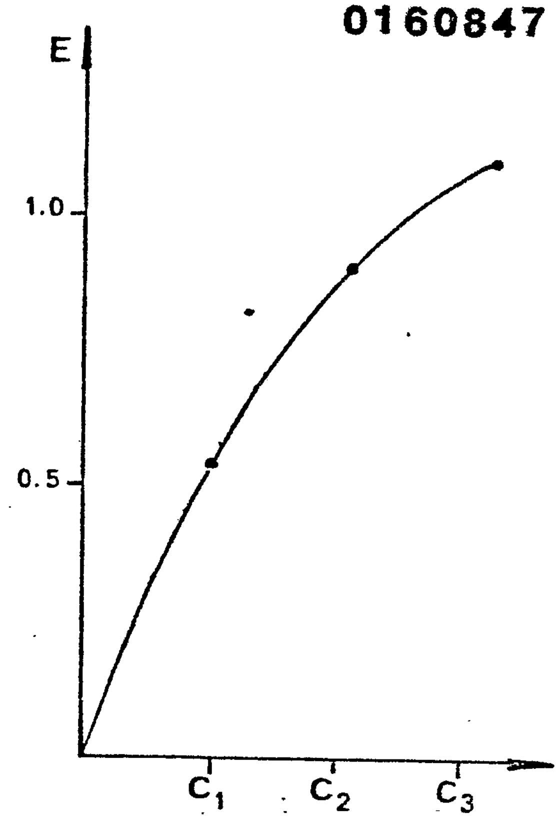


Abb. 2a

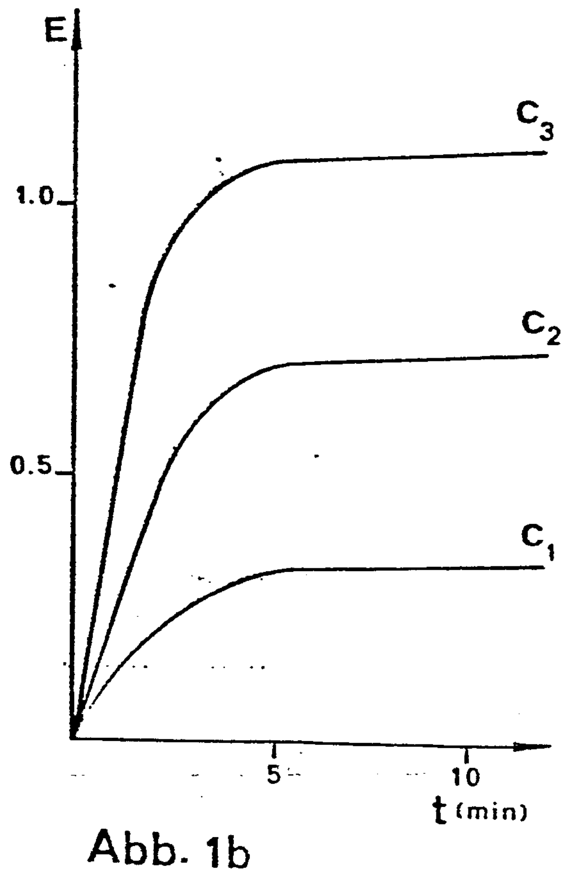


Abb. 1b

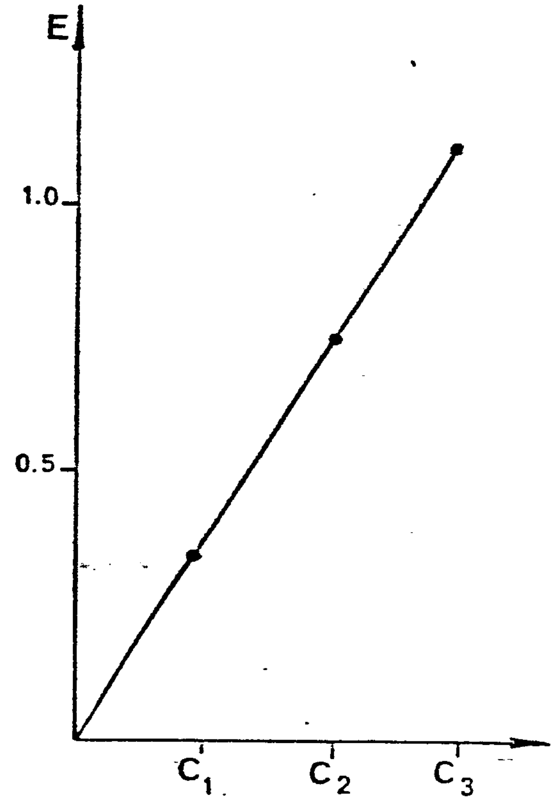


Abb. 2b

Europäisches Patentamt

# EUROPÄISCHER RECHERCHENBERICHT

0160847
Nummer der Anmeldung

EP 85 10 4140

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | | | G 01 N 33/52 //<br>C 12 Q 1/62<br>C 12 Q 1/54<br>C 12 Q 1/34 |
| Y | US-A-3 992 158 (E.P. PRZYBYLOWICZ et al.)<br>* Spalte 2, Zeile 65 - Spalte 3, Zeile 16; Spalte 3, Zeile 57 - Spalte 4, Zeile 30; Spalten 15-17; Beispiele 1, 3; Abbildung 4 * & DE-A-2 332 760 (Kat. D)<br>--- | 1,3,4,6 | |
| Y | GB-A-2 052 057 (FUJI PHOTO FILM CO., LTD.)<br>* Seiten 4-6; Beispiele 1-3 *<br>--- | 1,3,4,6 | |
| X | GB-A-1 519 465 (EASTMAN KODAK)<br>* Seite 2, Zeilen 19-54; Seite 11, Zeilen 75-87; Seiten 12, 13; Beispiele 1, 2 * & DE-A-2 532 918 (Kat. D)<br>--- | 1,6,9,10 | RECHERCHIERTE SACHGEBIETE (Int. Cl.4) |
| A | US-A-4 042 335 (P.L. CLEMENT)<br>* Abbildung 4; Spalte 5, Zeile 10 - Spalte 6, Zeile 6 *<br>--- | 1,6 | G 01 N 33/00<br>C 12 Q 1/00 |
| A | US-A-4 357 363 (Z.R. PIERCE et al.)<br>----- | | |

Der vorliegende Recherchenbericht wurde für alle Patentansprüche erstellt.

| Recherchenort | Abschlußdatum der Recherche | Prüfer |
|---|---|---|
| BERLIN | 16-07-1985 | GREEN C.H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument